# EUROPEAN PATENT SPECIFICATION

(11) **EP 1 902 722 B1**
(45) Date of publication and mention of the grant of the patent: **16.12.2020**
(21) Application number: 06780945.9
(22) Date of filing: 11.07.2006
(51) Int. Cl.: A61K 36/61, A61P 3/00, A61P 7/00

(54) **AGENT FOR ELEVATING ADIPONECTIN CONCENTRATION**
WIRKSTOFF ZUM ERHÖHEN DER ADIPONEKTINKONZENTRATION
AGENT POUR L'AUGMENTATION DE LA CONCENTRATION EN ADIPONECTINE

(30) Priority: 12.07.2005 JP 2005202691
(43) Date of publication of application: 26.03.2008
(73) Proprietor: Kabushiki Kaisha Yakult Honsha, Tokyo 105-8660 (JP)
(72) Inventor: TAKAMIZAWA, Kotaro, Minato-ku, Tokyo, 105-8660 (JP); MAKINO, Kumiko, Minato-ku, Tokyo, 105-8660 (JP); DEGUCHI, Yoriko, Minato-ku, Tokyo, 105-8660 (JP); ASANO, Tsuguyoshi, 120-0005 (JP); TSUJI, Atsushi, Chiba-shi, Chiba, 260-0844 (JP)
(74) Representative: Hartz, Nikolai
(86) International application number: PCT/JP2006/313723
(87) International publication number: WO 2007/007732

(56) References cited:
- WO-A1-01/66714
- JP-A- H0 640 929
- JP-A- H1 175 770
- JP-A- 2000 273 048
- JP-A- 2004 075 571
- DEGUCHI Y ET AL: "EFFECTS OF EXTRACT OF GUAVA LEAVES ON THE DEVELOPMENT OF DIABETES IN THE DB/DB MOUSE AND ON THE POSTPRANDIAL BLOOD GLUCOSE OF HUMAN SUBJECTS", NIPPON NOGEI KAGAKUKAISHI - JOURNAL OF THE AGRICULTURAL CHEMICAL SOCIETY OF JAPAN, NIPPON NOGEI KAGAKUKAI, vol. 72, no. 8, 1 January 1998 (1998-01-01), pages 923-931, XP002947275, TOKYO, JP ISSN: 0002-1407
- DIEZ J J ET AL: "The role of novel adipocyte-derived hormone adiponectin in human disease", EUROPEAN JOURNAL OF ENDOCRINOLOGY, vol. 148, 1 March 2003 (2003-03-01), pages 293-300, XP002993956, SCANDINAVIAN UNIVERSITY PRESS ISSN: 0804-4643, DOI: 10.1530/EJE.0.1480293
- KADOWAKI TAKASHI ET AL: "Adiponectin and adiponectin receptors", ENDOCRINE REVIEWS, vol. 26, no. 3, May 2005 (2005-05), pages 439-451, XP002645890, ISSN: 0163-769X
- DEGUCHI YORIKO ET AL: "Anti-hyperglycemic and anti-hyperlipidemic effects of guava leaf extract.", NUTRITION & METABOLISM, vol. 7, 9, 2010, pages 1-10, XP002645891, ISSN: 1743-7075
- GUTIERREZ ET AL: "Psidium guajava: A review of its traditional uses, phytochemistry and pharmacology", JOURNAL OF ETHNOPHARMACOLOGY, vol. 117, no. 1, 3 February 2008 (2008-02-03), pages 1-27, XP022558823, ELSEVIER SCIENTIFIC PUBLISHERS LTD, IE ISSN: 0378-8741
- WANG A ET AL: "Up-regulation of adiponectin by resveratrol: The essential roles of the Akt/FOXO1 and amp-activated protein kinase signaling pathways and DsbA-L", JOURNAL OF BIOLOGICAL CHEMISTRY, vol. 286, no. 1, 7 January 2011 (2011-01-07), pages 60-66, XP002645892, 10.1074/JBC.M110.188144 ISSN: 0021-9258, DOI: 10.1074/JBC.M110.188144
- ASANO T. ET AL.: 'Tonyobyo Kanja ni Taisuru Gaba-ha Inryo (Bansoreicha) no Rinsho Koka' JAPANESE JOURNAL OF NUTRITIONAL ASSESSMENT vol. 22, no. 2, 15 April 2005, pages 185 - 189, XP003007261
- ISHIBASHI G. ET AL.: 'Gaba no Ha Chushutsu Extract ga Rat Shishitsu Taisha ni Oyobosu Eikyo' JOURNAL OF HOME ECONOMICS OF JAPAN vol. 39, no. 4, 1988, pages 265 - 269, XP003007262
- UENO Y. ET AL: 'Adiponectin no Hatsugen Teika o Yokusei suru Shokuhin Inshi no Kento' ANNUAL MEETING OF JSBBA KOEN YOSHISHU vol. 2005, 05 March 2005, page 285, XP003007263

## Description

### Technical Field

The present invention relates to an agent for elevating adiponectin level (hereinafter may be referred to as an "adiponectin level elevating agent"), which is or contains guava leaf extract as an active ingredient for use in specific medical treatments and preventions.

### Background Art

Guava leaf extract has long been known to exhibit effects on, for example, diabetes, hypertension, constipation, obesity, and diarrhea, and is used for health-maintenance beverages as is or after being appropriately processed. Guava (*Psidium guajava* Linn.) is a native of tropical and subtropical regions, and is an evergreen species of Myrtaceae (*Psidium*)*.* Guava leaves are used for, for example, health-maintenance beverages as described above, whereas guava fruits are eaten raw or used for, for example, juice products.

In recent years, pharmacological effects of guava leaf extract have become of interest, and guava leaf extract has been proposed for various actions and applications, including a diet food having α-amylase inhibitory effect (Patent Document 1), a viral infection preventive/therapeutic agent (Patent Document 2), a lipid peroxide production inhibitor (Patent Document 3), a renal disease preventive/therapeutic agent (Patent Document 4), a glycation inhibitor (Patent Document 5), and a lipase inhibitor (Patent Document 6).

In accordance with recent development of molecular biology, various factors associated with human diseases have been elucidated. Particularly, adiponectin, which is an adipose-tissue-specific hormone factor, has been shown to exhibit, for example, insulin sensitivity-enhancing effect and anti-arteriosclerotic effect, and "hypoadiponectinemia" has been elucidated to be a fundamental pathological condition which causes the onset of diseases such as diabetes and arteriosclerosis (Non-Patent Document 1). In addition, adiponectin has been reported to exhibit therapeutic and preventive effects on various cancers, cardiac hypertrophy, intestinal polyp, infection, fibrosis, and inflammatory disease (Patent Document 7); therapeutic effect on acute wounds (Patent Document 8); preventive and ameliorative effects on hypertension (Patent Document 9); and preventive and therapeutic effects on cirrhosis and chronic hepatitis (Patent Document 10). Deguchi et al., J. AGRICULT. CHEM. SOC. JAPAN, vol. 72, no. 8, 1 January 1998, pages 923-931 discloses that a hot water extract of guava leafs has an inhibitory effect on several sugar degrading enzymes (maltase, sucrase, α-amylase) and reduces blood glucose levels in mice, which are treated with said extract. Diez. et al.,EUROP. J. ENDOCRIN., vol. 148, 1 March 2003, pages 293-300 reviews the role of adiponectin in human diseases, especially in diabetes and obesity. That is, adiponectin has been shown to correct abnormal glucose or lipid metabolism, and to be associated with various diseases.

Therefore, from the viewpoints of treatment, amelioration, and prevention of such various diseases, elevating blood adiponectin level or suppressing reduction in blood adiponectin level is very important, and thus demand has arisen for a highly safe substance which exhibits the effect of elevating blood adiponectin level, and which can be consumed over a long period of time.

However, in relation to a composition which exhibits the effect of elevating blood adiponectin level, only fermented tea extract has been reported (Patent Document 11); i.e., there is virtually no alternative. Meanwhile, guava leaf extract has not yet been reported to elevate blood adiponectin level.
Patent Document 1: Japanese Patent No. 2670742
Patent Document 2: JP-A-2000-273048
Patent Document 3: JP-A-1999-75770
Patent Document 4: Japanese Patent No. 3625900
Patent Document 5: JP-A-2004-250445
Patent Document 6: JP-A-2000-103741
Patent Document 7: JP-A-2004-345968
Patent Document 8: JP-A-2004-331590
Patent Document 9: JP-A-2004-275041
Patent Document 10: JP-A-2002-363094
Patent Document 11: JP-A-2004-315379

Non-Patent Document 1: Molecular Medicine, Vol. 39, No. 4, 416-423 (2002)

### Disclosure of the Invention

### Problems to be Solved by the Invention

**In view of the foregoing, an object of the present invention is to provide a highly safe medicament or food or beverage which exhibits the effect of elevating blood adiponectin level, and which allows intake thereof over a long period of time.**

### Means for Solving the Problems

**In order to solve the aforementioned problems, the present inventors have conducted extensive studies, and as a result have found that guava leaf extract exhibits the effect of elevating blood adiponectin level, and causes no side effects even when consumed routinely. The present invention has been accomplished on the basis of this finding.**

**Accordingly, the present invention provides an adiponectin level elevating agent** for use in a method of treatment or prevention of hypoadiponectinemia, said agent comprising a guava leaf extract as an active ingredient, wherein the guava leaf extract is obtained from guava leaves through extraction with water and/or a hydrophilic solvent.

The present invention also provides a guava leaf extract for use in a method of elevating the level of adiponectin in the treatment or prevention of hypoadiponectinemia, wherein the guava leaf extract is obtained from guava leaves through extraction with water and/ or a hydrophilic solvent.

The present invention also provides a guava leaf extract for use in a method of treatment or prevention of hypoadiponectinemia, wherein the guava leaf extract is obtained from guava leaves through extraction with water and/ or a hydrophilic solvent.

### Effects of the Invention

Guava leaf extract exhibits excellent adiponectin level elevating effect. Also, guava leaf extract exhibits a high level of safety, since it has long been used for, for example, health-maintenance beverages, and has been consumed as a dietary material for a long period of time. Therefore, the adiponectin level elevating agent for use according to the present invention can be widely employed for, for example, the treatment, amelioration, or prevention of diseases associated with adiponectin, namely hypoadiponectinemia. In addition, the adiponectin level elevating agent has few side effects, and exhibits safety. Best Modes for Carrying Out the Invention

The present invention employs leaves of guava (*Psidium guajava* Linn.). The guava leaf extract employed in the present invention is prepared by subjecting guava leaves to extraction with water and/or a hydrophilic organic solvent. Guava leaf extract is more preferable than guava leaf itself, from the viewpoints of potent adiponectin level elevating effect, and excellent taste.

Raw guava leaves or dried guava leaves may be subjected to extraction, and such guava leaves may be cut into pieces of an appropriate size (e.g., 3 mm to 5 mm) before use. Alternatively, dried and roasted guava leaves may be subjected to extraction. Employment of roasted guava leaves is preferred, from the viewpoint of improvement of flavor.

Examples of solvents which may be employed for extraction include water; lower alcohols such as methanol, ethanol, propanol, and butanol; esters such as ethyl acetate; glycols such as ethylene glycol, butylene glycol, propylene glycol, 1,3-butylene alcohol, and glycerin; ethers such as diethyl ether and petroleum ether; hydrophilic solvents such as acetone and acetic acid; and hydrocarbons such as benzene, hexane, and xylene. Water and/or hydrophilic solvents are preferred. These solvents may be employed singly or in combination of two or more kinds.

The weight of a solvent employed for extraction is preferably 1 to 100 times, particularly preferably 2 to 40 times of the dry weight of the plant (guava leaves).

Extraction may be performed through a generally employed method. Examples of the extraction method include a method in which guava leaves are immersed in a solvent; and a method in which guava leaves are stirred in a solvent under heated conditions (at ambient temperature to the boiling point of the solvent). Extraction may be performed by means of, for example, an autoclave for extraction.

Extraction conditions vary depending on the form of raw material or the type of a solvent employed. For example, extraction is performed at ambient pressure or under pressurized conditions (i.e., at a pressure of 1 atm to 2 atm) and at room temperature or under heated conditions. In the case of hot water extraction, for example, extraction is performed under heated conditions (at 50 to 130°C) for one minute to two hours. When the aforementioned extraction method is performed, bacteria which are probably present on guava leaves (i.e., raw material); for example, heat-resistant sporeformers, can be eliminated, which is preferred from the viewpoints of hygiene (i.e., no concern of bacterial and fungous contaminations) and a high level of safety.

Extraction may be performed under high pH conditions by addition of an alkali (e.g., sodium bicarbonate) to an extraction solvent, or under mild acidic conditions by addition of a dilute mineral acid (e.g., dilute hydrochloric acid) or an organic acid (e.g., succinic acid, citric acid, lactic acid, malic acid, or tartaric acid).

In the present invention, the thus-obtained guava leaf extract may be employed as is. However, before use, the thus-obtained extract may be subjected to separation through a customary method, and, if necessary, impurities may be removed. Alternatively, before use, the thus-obtained extract may be concentrated by means of, for example, a vacuum concentrator for removal of an extract solution, or may be powdered through freeze-drying or a similar technique.

Alternatively, before use, the above-obtained extract may be purified through an appropriate purification treatment (e.g., chromatography treatment). No particular limitation is imposed on, for example, the purification level or use form of the extract.

Guava leaf extract employed in the present invention may be a mixture of two or more extract products obtained through, for example, different extraction methods.

The thus-obtained guava leaf extract contains a high concentration of a naturally occurring active ingredient contained in guava leaves. When the guava leaf extract is administered to a living organism, although the mechanism of action of the extract is unknown, the extract exhibits the effect of elevating adiponectin level in, for example, blood, organs, tissues, or cells. Therefore, the guava leaf extract can be employed as an adiponectin level elevating agent; in particular, a blood adiponectin level elevating agent. The adiponectin level elevating agent for use according to the present invention, which contains the extract as an active ingredient, can be employed for the treatment, amelioration, or prevention of diseases associated with adiponectin, namely hypoadiponectinemia Also, the adiponectin level elevating agent for use according to the present invention can be employed for the prevention or amelioration of the same disease, namely hypoadiponectinemia.

The adiponectin level elevating agent of the present invention may be administered orally or parenterally. However, the agent is preferably administered orally. The agent may be administered in the form of a common pharmaceutical product by mixing the active ingredient with a non-toxic solid or liquid carrier for medicaments which is suitable for various administration methods (e.g., oral administration, intrarectal administration, and injection).

Examples of such a pharmaceutical product include solid products such as tablets, granules, powder, and capsules; liquid products such as solution, suspension, and emulsion; and freeze-dried products. Such a product may be prepared through pharmaceutically customary means. Examples of the aforementioned non-toxic carrier for medicaments include glucose, lactose, sucrose, starch, mannitol, dextrin, fatty acid glyceride, polyethylene glycol, hydroxyethyl starch, ethylene glycol, polyoxyethylene sorbitan fatty acid ester, amino acid, gelatin, albumin, water, and saline. If necessary, a commonly used additive (e.g., a stabilizer, a humectant, an emulsifier, a binder, an isotonizing agent, or an excipient) may be appropriately added.

The adiponectin level elevating agent for use according to the present invention may be employed not only for the aforementioned pharmaceutical products, but also for, for example, foods and beverages. In such a case, the aforementioned guava leaf extract *per se,* a mixture of the extract and various nutritional ingredients, or a food or beverage containing the extract may be employed as a healthcare food or food material useful for the purpose of elevating adiponectin level, or for, for example, the prevention or amelioration of diseases associated with adiponectin, including diabetes, arteriosclerosis, various cancers, cardiac hypertrophy, intestinal polyp, infection, fibrosis, inflammatory disease, acute wounds, hypertension, cirrhosis, and chronic hepatitis. For example, an additive which can be used as a food or beverage may be added to the aforementioned guava leaf extract, and then the resultant mixture may be prepared into a form suitable for food (e.g., granules, grains, tablets, capsules, or paste) through commonly employed means so as to provide food products thereof. Alternatively, the guava leaf extract may be added to a variety of foods; for example, processed meat products such as ham and sausage; processed fish products such as *kamaboko* and *chikuwa*; bread; confectionery; butter; powdered milk; and fermented milk products. Alternatively, the guava leaf extract may be added to beverages, such as water, juice, milk, and soft drink.

One preferred food or beverage product is a guava leaf tea beverage containing guava leaf extract as is. When such a guava leaf tea beverage is produced, preferably, there is employed a guava leaf extract product prepared through, for example, the following procedure: guava leaves are dried, and then roasted at about 120°C for 10 to 20 minutes; the guava leaves are cut into pieces having a size of 3 to 5 mm, followed by extraction with water of 50 to 100°C for 1 to 60 minutes (preferably 3 to 25 minutes); and the resultant guava leaf extract is concentrated or diluted to an appropriate concentration (preferably, so that the tannin content is about 0.05 to about 0.1%).

Guava leaf extract, which is an active ingredient of the adiponectin level elevating agent for use according to the present invention, has conventionally been used for foods, and has been shown to be safe. Therefore, no strict limitation is imposed on the dose of guava leaf extract when it is employed as an adiponectin level elevating agent. However, the daily dose of guava leaf extract is preferably 150 mg to 2 g, particularly preferably 400 mg to 1.6 g, as reduced to dry solid content.

### Examples

The present invention will next be described in more detail by way of Examples (Production Examples and Test Examples).

### Production Example 1 Production of guava leaf tea beverage

Guava leaves (from China) were dried, roasted at 121°C for 15 minutes, and then cut into pieces having a size of about 5 mm. Guava leaf pieces (75 kg) were added to hot water of 95°C (1,500 kg), followed by extraction for five minutes. The resultant extract was cooled to 30°C or lower, and clarified through centrifugation, followed by dilution with water so that the tannin content was 0.06 to 0.07 wt.%, to thereby yield a guava leaf extract product. Sodium ascorbate (0.05 wt.%) was added to the extract product, to thereby produce a guava leaf tea beverage.

### Production Example 2 Production of guava leaf extract product 1

Water (2 L) was added to dry guava leaves (100 g), followed by extraction under heating at 80°C for 30 minutes, and then filtered through quadruple gauze. The resultant guava leaf extract was freeze-dried into a powdery product (yield: about 14%).

### Production Example 3 Production of guava leaf extract product 2

Dry guava leaves (25 g) was subjected to extraction with 50% aqueous ethanol (500 mL) at ambient pressure and room temperature for one week. The resultant guava leaf extract was filtered through gauze, and the filtrate was dried under reduced pressure, to thereby yield a powdery product (yield: about 30%).

### Test Example 1 Studies on effect of guava leaf extract in elevating adiponectin level

The effect of guava leaf extract on blood adiponectin level was studied by use of the guava leaf tea beverage produced in Production Example 1. Test was performed in 23 subjects with mild hyperlipemia (15 females and eight males), who had a total cholesterol level of 180 mg/dL or more including borderline as measured before the test. The guava leaf tea beverage produced in Production Example 1 (200 mL) was given to each of the subjects thrice a day for eight weeks. Before initiation of intake of the beverage (hereinafter may be referred to "beverage intake"), and in week 4 and week 8 after initiation of beverage intake, the subjects were subjected to, for example, biochemical blood analyses, urinalyses, and a medical examination. The subjects were requested to fill out dietary questionnaires at one week after initiation of beverage intake, between week 4 and week 5 after initiation of beverage intake, and at one week before completion of beverage intake.

Although seven of these subjects were prescribed Lochol (an antilipemic agent), Mevalotin (an HMG-CoA reductase inhibitor), Livalo (a hypercholesterolemia therapeutic agent), Cholebine (a cholesterol absorption inhibitor), or Epadel (a triglyceride-lowering agent), the type and dose of such a medicament were not changed during the course of intake of the guava leaf tea beverage.

Before initiation of beverage intake and in week 8 after initiation of beverage intake, the subjects were classified into three groups according to blood adiponectin level; i.e., a group of less than 4 µg/mL, a group of 4 µg/mL or more and less than 7 µg/mL, and a group of 7 µg/mL or more. The data are shown in Table 1.

**[Table 1]**

| | | | |
|---|---|---|---|
| Change in blood adiponectin level through intake of guava leaf tea beverage (1) | | | |

| Adiponectin level | <4 µg/mL | ≥4 µg/mL and <7 µg/mL | ≥7 µg/mL |
|---|---|---|---|
| Before beverage intake | 3 subjects | 9 subjects | 11 subjects |
| Week 8 after beverage intake | 2 subjects | 7 subjects | 14 subjects |

As shown in Table 1, in week 8 after initiation of beverage intake, subjects were shifted to a group of higher blood adiponectin level; i.e., an elevation in blood adiponectin level was observed as a whole. Particularly, nine of the 15 female subjects were found to have elevated blood adiponectin level. The average of blood adiponectin levels of all the subjects was elevated from 8.6 µg/mL (before initiation of beverage intake) to 8.9 µg/mL (in week 8 after initiation of beverage intake).

Table 2 shows blood adiponectin levels of subjects who had, before initiation of beverage intake, a body fat percentage falling within a range of obesity (female: >30%, male: ≥25%), the blood adiponectin levels being measured before initiation of beverage intake, and in week 4 and week 8 after initiation of beverage intake. Table 2 also shows blood adiponectin levels of subjects who had, before initiation of beverage intake, an HbA_{1c} (glycosylated hemoglobin) level falling within a diabetic range (i.e., 6.5% or more).

**[Table 2]**

| | | | | | |
|---|---|---|---|---|---|
| Change in blood adiponectin level through intake of guava leaf tea beverage (2) | | | | | |

| Items | | n | Blood adiponectin level (µg/mL) | | |
|---|---|---|---|---|---|
| | | | Before beverage intake | Week 4 after beverage intake | Week 8 after beverage intake |
| Body fat percentage | Female: 30% or more | 13 | 8.5 ± 3.0 | 8.3 ± 3.2 | 8.7 ± 3.8 |
| | Male: 25% or more | 3 | 4.2 ± 1.0 | 4.4 ± 1.8 | 6.2 ± 2.8 |
| HbA_{1c} | 6.5% or more | 9 | 5.7 ± 1.8 | 5.7 ± 2.3 | 6.8 ± 3.2* |

| | | | | | |
|---|---|---|---|---|---|
| *: P = 0.08 (comparison with values before beverage intake) | | | | | |

As shown in Table 2, in week 8 after initiation of beverage intake, an elevation in adiponectin level was observed in the subjects who had, before initiation of beverage intake, a body fat percentage falling within a range of obesity. Similarly, in week 8 after initiation of beverage intake, an elevation in adiponectin level was observed in the subjects who had, before initiation of beverage intake, an HbA_{1c} (glycosylated hemoglobin) level falling within a diabetic range.

In addition, the following data were obtained.
1. In week 8 after initiation of beverage intake, the female subjects showed downward trends in total blood cholesterol level (p = 0.07) and in LDL-cholesterol level (p = 0.05). In contrast, no change in HDL-cholesterol level was observed.
2. Subjects who had, before initiation of beverage intake, a high total cholesterol level (i.e., 220 mg/dL or more) showed a downward trend in total cholesterol level (p = 0.06) in week 4 after initiation of beverage intake, and a significant decrease in total cholesterol level (p < 0.05) in week 8 after initiation of beverage intake. The subjects also showed a downward trend in LDL-cholesterol level (p = 0.06). These trends were observed remarkably in female subjects.
3. In week 8 after initiation of beverage intake, HbA_{1c} level was significantly lowered (p < 0.05).
4. Neither abnormality nor great change was observed in other blood test data and urine test data. Adverse events (e.g., diarrhea, constipation, anorexia, and discomfort) did not occur, and no great change was observed in dietary contents during the course of beverage intake.

As is clear from the aforementioned data, when a human subject takes guava leaf extract, the subject shows an elevation in blood adiponectin level. Conceivably, the effect of elevating blood adiponectin level is attributed to intake of guava leaf extract, since no great change was observed in dietary contents during the course of intake of the guava leaf beverage, and the type and dose of medicaments prescribed before beverage intake were not changed. In addition, no adverse events occurred during the course of beverage intake, and no abnormality was observed in blood test data and urine test data; i.e., guava leaf extract was found to be safe. As has been pointed out, particularly when human blood adiponectin level is 4 µg/mL or less, the risk of coronary artery diseases is increased (Bio Clinica, 19, 18-30, 2004). In the aforementioned test, the number of human subjects having a blood adiponectin level of less than 4 µg/mL was reduced in week 8 after initiation of beverage intake, and the average of blood adiponectin levels of all the subjects was elevated. These data indicate that intake of guava leaf extract is effective for living organisms. In addition, subjects who had, before initiation of beverage intake, a body fat percentage falling within a range of obesity or an HbA_{1c} level falling within a diabetic range showed an elevation in blood adiponectin level through beverage intake, which indicates that guava leaf extract is particularly effective for the prevention or amelioration of metabolic syndrome.

### Formulation Example 1 Production of tablet

The following ingredients were mixed according to the following formulation, and the mixture was subjected to granulation, drying, and granule size regulation, followed by tableting for production of tablets.

| (Formulation) | (mg) |
|---|---|
| Extract product of Production Example 3 | 40 |
| Microcrystalline cellulose | 100 |
| Lactose | 80 |
| Magnesium stearate | 0.5 |
| Methylcellulose | 12 |

### Formulation Example 2 Production of soft drink

The following ingredients were mixed through a customary method according to the following formulation, and then homogenized, to thereby yield a soft drink. The thus-obtained soft drink was charged into a brown bottle, and then the bottle was sealed with an aluminum cap, followed by thermal treatment.

| (Formulation) | (g) |
|---|---|
| Extract product of Production Example 2 | 0.8 |
| Perfume | 0.8 |
| Water | 100 |
| Reduced saccharified starch | 24 |
| Fructose | 18 |

### Formulation Example 3 Production of fermented milk product

Three % glucose was added to 15% skim milk, followed by sterilization at 120°C for three seconds. Thereafter, a seed culture of *Lactobacillus casei* YIT9029 (1%) was inoculated to the resultant mixture, followed by culturing at 37°C to a pH of 3.6, to thereby yield 210 g of a yogurt base. Separately, sugar (97 g), iron citrate (0.2 g), and the extract product of Production Example 2 (5 g) were dissolved in water, followed by addition of water to a total amount of 790 g. The resultant solution was sterilized at 110°C for three seconds, to thereby yield a syrup. The above-obtained yogurt base and the syrup were mixed together, and a perfume (1 g) was added to the mixture. Thereafter, the mixture was homogenized at 15 MPa, and then charged into a container, to thereby yield a fermented milk product.

## Claims

1. A guava leaf extract for use in a method of treatment or prevention of hypoadiponectinemia, wherein the guava leaf extract is obtained from guava leaves through extraction with water and/or a hydrophilic solvent.

2. A guava leaf extract for use in a method of elevating the level of adiponectin in the treatment or prevention of hypoadiponectinemia, wherein the guava leaf extract is obtained from guava leaves through extraction with water and/or a hydrophilic solvent.

3. An adiponectin level elevating agent for use in a method of treatment or prevention of hypoadiponectinemia, said agent comprising a guava leaf extract as an active ingredient, wherein the guava leaf extract is obtained from guava leaves through extraction with water and/or a hydrophilic solvent.

4. The guava leaf extract for the use according to claim 1 or 2, wherein a high total cholesterol level of 220 mg/dl or more is treated in subjects.

5. The guava leaf extract for the use according to claim 1 or 2 in human subjects having a blood adiponectin level of less than 4 µg/ml.

## Patentansprüche

1. Ein Guavenblattextrakt zur Verwendung in einem Verfahren zur Behandlung oder Vorbeugung von Hypoadiponectinämie, wobei der Guavenblattextrakt aus Guavenblättern durch Extraktion mit Wasser und/oder einem hydrophilen Lösungsmittel erhalten wird.

2. Ein Guavenblattextrakt zur Verwendung in einem Verfahren zur Erhöhung des Adiponectinspiegels bei der Behandlung oder Vorbeugung von Hypoadiponectinämie, wobei der Guavenblattextrakt aus Guavenblättern durch Extraktion mit Wasser und/oder einem hydrophilen Lösungsmittel erhalten wird.

3. Ein Adiponectinspiegel-Erhöhungsmittel zur Verwendung in einem Verfahren zur Behandlung oder Vorbeugung von Hypoadiponectinämie, wobei das Mittel einen Guavenblattextrakt als aktiven Bestandteil umfasst, wobei der Guavenblattextrakt aus Guavenblättern durch Extraktion mit Wasser und/oder einem hydrophilen Lösungsmittel erhalten wird.

4. Der Guavenblattextrakt für die Verwendung nach Anspruch 1 oder 2, wobei ein hoher Gesamtcholesterinspiegel von 220 mg/dl oder mehr bei Subjekten behandelt wird.

5. Der Guavenblattextrakt zur Verwendung nach Anspruch 1 oder 2 bei Menschen mit einem Blutadiponectinspiegel von weniger als 4 µg/ml.

## Revendications

1. Extrait de feuilles de goyavier pour une utilisation dans une méthode de traitement ou de prévention de l'hypoadiponectinémie, dans lequel l'extrait de feuilles de goyavier est obtenu à partir de feuilles de goyavier par extraction avec de l'eau et/ou un solvant hydrophile.

2. Extrait de feuilles de goyavier pour une utilisation dans une méthode d'élévation du niveau d'adiponectine dans le traitement ou la prévention de l'hypoadiponectinémie, dans lequel l'extrait de feuilles de goyavier est obtenu à partir de feuilles de goyavier par extraction avec de l'eau et/ou un solvant hydrophile.

3. Agent élevant le niveau d'adiponectine pour une utilisation dans une méthode de traitement ou de prévention de l'hypoadiponectinémie, ledit agent comprenant un extrait de feuilles de goyavier en tant que principe actif, dans lequel l'extrait de feuilles de goyavier est obtenu à partir de feuilles de goyavier par extraction avec de l'eau et/ou un solvant hydrophile.

4. Extrait de feuilles de goyavier pour une utilisation selon la revendication 1 ou 2, dans lequel un niveau de cholestérol total élevé de 220 mg/dl est traité chez des sujets.

5. Extrait de feuilles de goyavier pour une utilisation selon la revendication 1 ou 2 sur des sujets humains ayant un niveau sanguin d'adiponectine inférieur à 4 µg/ml.
